# EUROPEAN PATENT SPECIFICATION

(11) **EP 0 922 048 B1**
(45) Date of publication and mention of the grant of the patent: **15.11.2000**
(21) Application number: 97940076.9
(22) Date of filing: 11.08.1997
(51) Int. Cl.: C07D 498/04, C07D 209/42

(54) **PROCESS FOR THE PREPARATION OF N- (1-n-BUTYL-4-PIPERIDINYL)METHYL] -3,4-DIHYDRO -2H-[1,3] OXAZINO[3,2-a] INDOLE-10-CARBOXAMIDE AND SALTS AND INTERMEDIATES IN THE PROCESS**
VERFAHREN ZUR HERSTELLUNG VON N- (1-n-BUTYL-4-PIPERIDINYL)METHYL] -3,4-DIHYDRO -2H-[1,3]OXAZINO[3,2-a]INDOL-10-CARBOXAMID UND SALZE SOWIE ZWISCHENPRODUKTE IN DEM VERFAHREN
PROCEDE POUR LA PREPARATION DE N- (1-n-BUTYL-4-PIPERIDINYL)METHYLE] -3,4-DIHYDRO -2H-[1,3] OXAZINO[3,2-A] INDOLE-10-CARBOXAMIDE ET SELS ET INTERMEDIAIRES UTILISES DANS CE PROCEDE

(30) Priority: 16.08.1996 GB 9617188; 11.09.1996 GB 9618968
(43) Date of publication of application: 16.06.1999
(73) Proprietor: SMITHKLINE BEECHAM PLC, Brentford, Middlesex TW8 9EP (GB)
(72) Inventor: FEDOULOFF, Michael, SmithKline Beecham Pharmaceut., Harlow, Essex CM19 5AW (GB); SMITH, Gillian Elizabeth, SmithKline Beecham Pharm, Harlow, Essex CM19 5AW (GB); GUEST, David William, SmithKline Beecham Pharm., Harlow, Essex CM19 5AW (GB); STRACHAN, John Bryce, SmithKline Beecham Pharm., Harlow, Essex CM19 5AW (GB)
(74) Representative: Garrett, Michael
(86) International application number: EP9704413
(87) International publication number: WO9807728

(56) References cited:
- WO-A-93/18036
- GASTER L M ET AL: "N-[(1-Butyl-4-piperidinyl)methyl]-3,4- dihydro-2H-[1,3]oxazino[3,2- a]indole- 10-carboxamide hydrochloride: the first potent and selective 5-HT4 receptor antagonist amide with oral activity." J. MED. CHEM. (JMCMAR,00222623);95; VOL.38 (24); PP.4760-3, SMITHKLINE BEECHAM PHARMACEUTICALS;NEW FRONTIERS SCIENCE PARK; HARLOW ESSEX; CM19 5AW; UK (GB), XP002049018
- WYMAN P A ET AL: "Azabicyclic Indole Esters as Potent 5-HT4 Receptor Atagonists" BIOORG. MED. CHEM. (BMECEP,09680896);96; VOL.4 (2); PP.255-61, SMITHKLINE BEECHAM PHARMACEUTICALS;ESSEX; CM19 5AW; UK (GB), XP002049019

## Description

This invention relates to a new synthetic process to a compound having pharmacological activity.

WO 93/18036 (SmithKline Beecham plc) describes compounds having 5-HT₄ receptor antagonist activity of formula (I), or a pharmaceutically acceptable salt thereof: 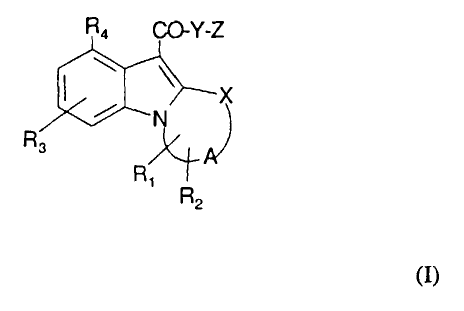 wherein
X is O, S, SO, SO₂, CH₂, CH or NR wherein R is hydrogen or C₁₋₆ alkyl;
A is a saturated or unsaturated polymethylene chain of 2 - 4 carbon atoms;
R₁ and R₂ are hydrogen or C₁₋₆ alkyl;
R₃ is hydrogen, halo, C₁₋₆ alkyl, amino, nitro or C₁₋₆ alkyl;
R₄ is hydrogen, halo, C₁₋₆ alkyl or C₁₋₆ alkoxy;
Y is O or NH;
Z is of sub-formula (a), (b) or (c): 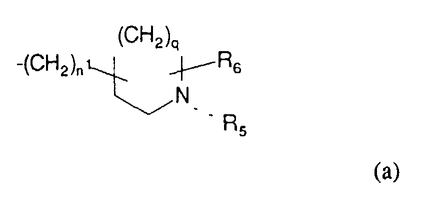 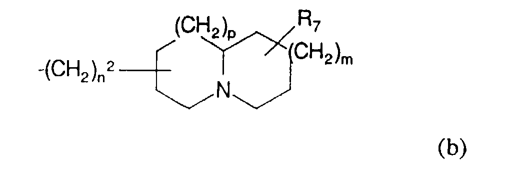 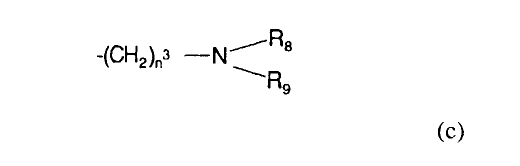
wherein
n¹ is 1, 2, 3 or 4; n² is 0, 1, 2, 3 or 4; n³ is 2, 3, 4 or 5;
q is 0, 1, 2 or 3; p is 0, 1 or 2; m is 0, 1 or 2;
R₅ is hydrogen, C₁₋₁₂ alkyl, aralkyl or R₅ is (CH₂)_{z}-R₁₀ wherein z is 2 or 3 and R₁₀ is selected from cyano, hydroxyl, C₁₋₆ alkoxy, phenoxy, C(O)C₁₋₆ alkyl, COC₆H₅, -CONR₁₁R₁₂, NR₁₁COR₁₂, SO₂NR₁₁R₁₂ or NR₁₁SO₂R₁₂ wherein R₁₁ and R₁₂ are hydrogen or C₁₋₆ alkyl; and
R₆, R₇ and R₈ are independently hydrogen or C₁₋₆ alkyl; and
R₉ is hydrogen or C₁₋₁₀ alkyl;
or a compound of formula (I) wherein the CO-Y linkage is replaced by a heterocyclic bioisostere;
having 5-HT₄ receptor antagonist activity.

Examples of alkyl or alkyl containing groups described herein include C₁, C₂, C₃, C₄, C₅, C₆, C₇, C₈, C₉, C₁₀, C₁₁ or C₁₂ branched, straight chained or cyclic alkyl, as appropriate. C₁₋₄ alkyl groups include methyl, ethyl, *n-* and iso-propyl, *n-, iso-, sec-* and *tert*-butyl. Cyclic alkyl includes cyclopropyl, cyclobutyl, cyclopentyl, cyclohexyl, cycloheptyl and cyclooctyl.

The pharmaceutically acceptable salts of the compounds of the formula (I) include acid addition salts with conventional acids such as hydrochloric, hydrobromic, boric, phosphoric, sulphuric acids and pharmaceutically acceptable organic acids such as acetic, tartaric, maleic, citric, succinic, benzoic, ascorbic, methanesulphonic, α-keto glutaric, α-glycerophosphoric, and glucose-1-phosphoric acids.

Examples of pharmaceutically acceptable salts include quaternary derivatives of the compounds of formula (I) such as the compounds quaternised by compounds Rₓ-T wherein Rₓ is C₁₋₆ alkyl, phenyl-C₁₋₆ alkyl or C₅₋₇ cycloalkyl, and T is a radical corresponding to an anion of an acid. Suitable examples of Rₓ include methyl, ethyl and n- and iso-propyl; and benzyl and phenethyl. Suitable examples of T include halide such as chloride, bromide and iodide.

Examples of pharmaceutically acceptable salts also include internal salts such as N-oxides.

The compounds of the formula (I), their pharmaceutically acceptable salts, (including quaternary derivatives and N-oxides) may also form pharmaceutically acceptable solvates, such as hydrates, which are included wherever a compound of formula (I) or a salt thereof is referred to.

Example 3 describes the hydrochoride salt of the compound of formula (I):
A is -CH₂-(CH₂)ᵣ-CH₂- wherein r is 1;
R₁ and R₂ are hydrogen;
R₃ is hydrogen;
R₄ is hydrogen;
Y is NH; and
Z is of sub-formula (a), and is of structure (i):

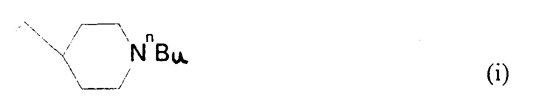

This compound is N-[(1-ⁿbutyl-4-piperidyl)methyl]-3,4-dihydro-2H-[1,3]oxazino[3,2-a]indole-10-carboxamide SB 207266, (the hydrochloride salt is SB 207266-A) which is being developed by SmithKline Beecham plc as the active ingredient in a medicament for treatment of irritable bowel syndrome.

Example 3 of WO 93/18036 describes a method of preparation of SB 207266-A from N-[(1-ⁿbutyl-4-piperidyl)methyl]indole-3-carboxamide (i.e. the compound corresponding to SB 207266, without the oxazino moiety), by reacting with N-chlorosuccinimide and 3-bromo-1-propanol, followed by treatment with sodium carbonate. N-[(1-ⁿbutyl-4-piperidyl)methyl]indole-3-carboxamide is prepared by coupling N-(1-ⁿbutyl-4-piperidyl)methylamine with a indole-3-carboxylic acid.

An alternative process for preparing SB 207266-A has now been discovered which involves the use of the N-(1-ⁿbutyl-4-piperidyl)methylamine intermediate at a later stage in the process thus resulting in an increased yield of SB 207266-A relative to the amount of this intermediate, which is relatively expensive to produce.

Accordingly, the present invention provides a process for the preparation of SB 207266 or a pharmaceutically acceptable salt thereof, which process comprises the reaction of of N-(1-ⁿbutyl-4-piperidyl)methylamine with a compound of formula (A): 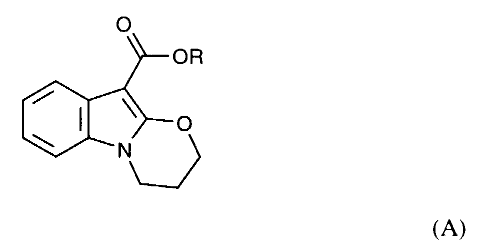 wherein R is alkyl, such as methyl or ethyl.

The compound of formula (A) wherein R is methyl is methyl 3,4-dihydro-2*H*-[1,3]-oxazino[3,2-*a*]indole-10-carboxylate.

The conditions and reagents for this reaction can be similar to those described in the literature.

Preferably, the reaction is catalysed by an aluminium or lithium based catalyst, more preferably trimethylaluminium. According to a preferable embodiment of the invention, a mixture of the amine and ester in a suitable solvent (eg toluene) is treated with a solution of trimethylaluminium in toluene or hexanes at ambient temperature. The resulting solution is then heated, preferably to reflux (112 °C ) for about four hours until the reaction is complete. The reaction is cooled to about 70°C and quenched by cautious addition of aqueous sodium hydroxide solution. The aqueous layer is separated and the mixture washed once more with caustic and twice with water, maintaining the temperature at about 70°C. The product is isolated as described in the attached Example.

Alternative catalysts include NaH₂Et₂Al which is used in a similar way to AlMe₃.

BuLi is also suitable but is used at lower temperatures and requires two equivalents of the base and two equivalents of the amine.

The mechanism of the reaction and role of the aluminium or lithium based catalyst is discussed in the references listed below:
- Use of AlMe₃:: Anwer Basha, Michael Lipton and Steven M. Weinreb, Tetrahedron Letters, 48, 4171, 1977.
- Use of NaH₂Et₂Al:: Tae Bo Sim and Nung Min Yoon, Synlett., 1994, 827
- Use of BuLi:: Kim-Wenn Yang, Joseph, G. Cannon and John G. Rose, Tetrahedron Letters, 21, 1791, 1970

The oxazinoindole compound of the formula (A) is preferably prepared from the corresponding indole by reaction with N-chlorosuccinimide and a 3-halopropanol, such as 3-chloropropanol or 3-bromopropanol followed by cyclisation of the intermediate (B) by treatment with base in a suitable solvent.

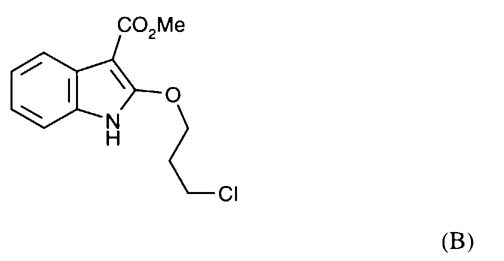

Suitable solvents for the cyclisation include acetone and toluene, and suitable bases include potassium carbonate, aqueous sodium hydroxide.

The use of aqueous sodium hydroxide solution, even at 80°C does not cause any significant hydrolysis of the ester.

In the case of toluene/aqueous sodium hydroxide, a phase transfer catalyst (eg tetrabutylammonium bromide) may be added, resulting in accelerating the reaction and allowing it to proceed at a lower temperature.

The intermediate (B) may either be used as a crude oil or isolated as a white crystalline solid and then cyclised in quantitative yield to give a solution of (A) in a suitable solvent (eg toluene) for coupling with the amine.

Compounds of the formula (A) and (B) are novel and form an aspect of the invention.

The following Example illustrates the invention. The following Description illustrates the preparation of an intermediate of formula (A).

### Example

### i) Preparation of N-[(1-butyl-4-piperidinyl)methyl]-3,4-dihydro-2H-[1,3]-oxazino[3,2-a]indole-10-carboxamide [SB-207266]

***Method A*** Toluene (85L) was azeotropically dried in an argon purged reactor, cooled to 10°C and a solution of trimethylaluminium in toluene (18.57kg, 16.7% w/w, 43 mole) added. To this at 20 to 24°C was added a solution of 1-*n*-butyl-4-piperidinylmethylamine (7.39kg, 99.4% pure, 42.7mole) in toluene (22L) over 43 minutes. Methyl 3,4-dihydro-2*H*-[1,3]-oxazino[3,2-*a*]indole-10-carboxylate (9.65kg, 98.9% pure, 41.3 mole) was added in one portion and the reaction heated to reflux at 112°C for 4 hours 10 minutes after which time the reaction was judged to be complete by HPLC analysis. 10% Sodium hydroxide solution (52.2L), prepared from 32% ww sodium hydroxide (24L) and water (80L), was added cautiously over 16 minutes at about 60 to 70°C. The resulting mixture was heated to 70 to 80°C and the aqueous layer separated. The toluene layer was washed with 10% sodium hydroxide (52.2L) followed twice by water (29L each wash). The toluene layer was cooled and diluted with hexane fraction (133L) to crystallise the product. After cooling to about 2°C overnight the product was collected by filtration, washed on the filter with hexane (21L) and dried *in vacuo* at 40°C overnight to give SB-207266 batch 207266-HP8 (12.26kg, 94.5% pure, 75.9%).

***Method B*** 1.6M Butyllithium in hexane (1.4ml) was added to toluene (2ml) at-10°. A solution of 1-*n*-butyl-4-piperidinylmethylamine (0.38g) in toluene (3ml) was added and the mixture was stirred for 5 mins. A solution of methyl 3,4-dihydro-2*H*-[1,3]-oxazino[3,2-*a*]indole-10-carboxylate (0.23g) in hot toluene (5ml) was added and the mixture was stirred at -10° for 5 mins. The mixture was diluted to 1000ml with acetonitrile:water and relative assay of the solution showed an SB-207266 content of 343mg (93% yield).

***Method C*** A mixture of methyl 2-(3-chloropropoxy)-indole-3-carboxylate (100g, 0.37mol), aqueous sodium hydroxide solution (38ml, 10.8M, 0.41mol), water (38ml) and tetrabutylammonium bromide (6.0g, 0.019mol) in toluene (1000ml) was stirred at 50 - 60°C for about one hour. Water (120ml) was added and the aqueous layer was removed. The organic layer was washed with water (120ml) and dried by azeotropic distillation of toluene (250ml) giving a dry solution of methyl 3,4-dihydro-2*H*-[1,3]-oxazino[3,2-a]indole-10-carboxylate in toluene. This solution was cooled to ambient temperature and treated sequentially with a solution of 1-butyl-4-piperidinylmethylamine (66.8g, 0.39mol) in toluene (200ml) followed by a solution of trimethylaluminium in toluene (196ml, 2.0M, 0.39mol). The mixture was heated to reflux and stirred for three hours. The reaction was quenched by cautious addition of aqueous sodium hydroxide solution (460ml, 10% w/v) and then washed once with aqueous sodium hydroxide solution (460ml, 10% w/v) and twice with water (275ml each wash) while maintaining the temperature at about 70°C. Toluene (200ml) was added and the resulting solution was dried by azeotropic distillation of toluene (200ml) at about 55°C under reduced pressure. Hexane (1400ml) was added and the resulting slurry cooled to about 0 - 5°C for about one hour. The solid was isolated by filtration and dried *in vacuo* to give the product, N-[(1-butyl-4-piperidinyl)methyl]-3,4-dihydro-2*H*-[1,3]-oxazino[3,2-a]indole-10-carboxamide, (114.7g, 83%) as a white crystalline solid.

### ii) Preparation of N-[(1-butyl-4-piperidinyl)methyl]-3,4-dihydro-2H-[1,3]-oxazino[3,2-a]indole-10-carboxamide hydrochloride [SB-207266-A]

***Method A*** N-[(1-Butyl-4-piperidinyl)methyl]-3,4-dihydro-2*H*-[1,3]-oxazino[3.2-a]indole-10-carboxamide (SB-207266) (12.26kg 94.5% pure, 31.35 moles) was dissolved in acetone (70.5L) at 41°C. Anhydrous HCl in propan-2-ol (8.98L, 3.86 molar, 34.7 moles), made by dissolving HCl gas (3.1kg) in propan-2-ol (20L), was added over 8 minutes allowing the temperature to rise to 57°C. The mixture was cooled to 4°C and stirred at 2 to 4°C for 2 hours. The precipitate was filtered off. washed with cold acetone (25L) and dried at atmospheric pressure at 40 to 50°C for 17 hours to give the crude product as a white solid (12.94kg; 96.1%). The crude product (12.94kg) was dissolved in hot ethanol (107L) and filtered through celite, washing the filter bed with further hot ethanol (18L). The filtrate was heated to 75°C and hot filtered hexane (68L) was added. The mixture was cooled to 19°C over about 4 hours and then to 4°C and stirred overnight at 1 °C. The white solid was filtered off, washed with a 1:1 mixture of cold ethanol/hexane (27L) and dried *in vacuo* at 50°C for 23 hours to give SB-207266A (12.36kg, 96.2% from SB-207266). This was milled in an Apex Comminuting mill through a 0.125 inch x 0.125 inch square mesh at medium speed and with hammers forward. 12.3kg (95.8% from SB-207266) was isolated as a fine, homogeneous white powder.

***Method B*** N-[(1-Butyl-4-piperidinyl)methyl]-3,4-dihydro-2*H*-[1,3]-oxazino[3,2-*a*]indole-10-carboxamide (SB-207266) (100g, 0.27mol) was dissolved in ethanol (870ml) and the resulting solution filtered to remove particulates. Anhydrous HCl in ethanol (83ml, 3.6M, 0.30mol) was added causing the product to precipitate out of solution. The slurry was heated to redissolve the solid and hexane (550ml) was added. After cooling to room temperature, the mixture was cooled to 0 - 5°C and stirred at that temperature for about two hours. The solid was isolated by filtration and dried *in vacuo* at about 40°C to give the product, N-[(1-butyl-4-piperidinyl)methyl]-3,4-dihydro-2*H*-[1,3]-oxazino[3,2-*a*]indole-10-carboxamide hydrochloride, (102.8g) in 94% yield.

### Description

**Preparation of methyl-3,4 dihydro-2H-[1,3]-oxazino[3,2-*a*]indole-10-carboxylate** ***Method A*** A solution of 3-chloropropanol (14.74kg, 98.4% pure, 153.4 mole) in dichloromethane (67L) was cooled to -17°C. In a second vessel dichloromethane (68L), methyl indole-3-carboxylate (13.5kg, 99.8% pure, 76.9 mole) and 1,4-diazabicyclo[2.2.2]octane (4.75kg, assumed 100% pure, 42.3 mole) were cooled to 0°C. N-Chlorosuccinimide (11.3kg, 99.5% pure, 84.2 mole) was added to the second vessel and stirred at 0°C for 10 minutes. In the meantime methane sulphonic acid (0.59L, 99.7% pure, 6.14 mole) was added to the first vessel. The solution in the second vessel was added to the first vessel over 49 minutes at -15 to 3°C and the resulting mixture stirred for a further 31 minutes at -5 to 0°C. 10% Sodium carbonate solution (147L), made from sodium carbonate (42.2kg, 398 mole) and process water (422L), was added over 14 minutes and stirred. The organic layer was separated and washed twice with 10% sodium carbonate solution (147L each wash). The organic layer was dried over anhydrous sodium sulphate, filtered and concentrated under reduced pressure below 30°C. The concentrate was dissolved in acetone (101L) at about 18°C and potassium carbonate (14.9kg) added. The mixture was stirred at 18 to 28°C for 18 hours. Analysis of the reaction showed it to be complete. The inorganic salts were filtered off, the filtrate concentrated under reduced pressure below 30°C and dissolved in dichloromethane (101L). The dichloromethane solution was washed twice with 5% sodium bicarbonate solution (85L each wash), made from sodium bicarbonate (8.3kg) and water (167L) and dried over anhydrous sodium sulphate. After filtration the filtrate was concentrated under reduced pressure to a base temperature of about 95°C and diluted with toluene (12L). The toluene solution was cooled, causing the product to crystallise and cooling continued to about 0°C overnight. The product was collected by filtration, washed on the filter with cold (0°C) toluene (7L) and dried *in vacuo* at 30°C for 21 hours to give the title compound (9.654kg, 98.9% pure, 53.7%).

***Method B*** A solution of 3-chloropropanol (142.47g, 1.51 mole) in dichloromethane (1200ml) was cooled to -20°C. In a second vessel dichloromethane (1300ml), methyl indole-3-carboxylate (240.0g, 1.37 mole) and 1,4-diazabicyclo[2.2.2]octane (84.52g 0.75 mole) were cooled to 0°C. N-Chlorosuccinimide (201.22g 1.51 mole) was added to the second vessel and stirred at 0°C for 10 minutes. In the meantime methane sulphonic acid (10.56ml) was added to the first vessel. The solution in the second vessel was added to the first vessel keeping the temperature below about 0°C, and the resulting mixture stirred for a further 2.5 hours at -5 to 0°C. 10% Sodium carbonate solution (2500ml) was added and stirred. The organic layer was separated and washed twice with 10% sodium carbonate solution (2500ml each wash). The organic layer was dried over anhydrous sodium sulphate, filtered and concentrated under reduced pressure. The concentrate was triturated with ethyl acetate (120ml) and the mixture stirred at 0°C for about 1 hour. The resulting solid was filtered, washed a small quantity of ethyl acetate and dried under vacuum to give *methyl 2-(3-chloropropoxy)-indole-3-carboxylate* (202.5g) as a white crystalline solid in 55% yield.

A mixture of methyl 2-(3-chloropropoxy)-indole-3-carboxylate (81.5g, 0.304 mole), aqueous sodium hydroxide solution (31ml, 10.8M, 0.335 mole), water (31ml) and tetrabutylammonium bromide (4.9g, 0.015 mole) in toluene (815ml) was stirred at 50 - 60°C for about 45 minutes. The aqueous layer was removed and the organic layer washed twice with water (100ml each wash). The resulting toluene solution was dried by azeotropic distillation of solvent (265ml) under reduced pressure (60°C, 160mbar) giving a dried solution of the title compound in toluene.

***Method C*** A solution of 3-chloropropanol (142.47g, 1.51 mol) in dichloromethane (1200ml) was cooled to -20°C. In a second vessel dichloromethane (1300ml), methyl indole-3-carboxylate (240.0g, 1.37 mol) and 1,4-diazabicyclo[2.2.2]octane (84.52g 0.75 mol) were cooled to 0°C. N-Chlorosuccinimide (201.22g 1.51 mole) was added to the second vessel and stirred at 0°C for 10 minutes. In the meantime methane sulphonic acid (10.56ml) was added to the first vessel. The solution in the second vessel was added to the first vessel keeping the temperature below about 0°C, and the resulting mixture stirred for a further 2.5 hours at -5 to 0°C. 10% Sodium carbonate solution (1250ml) was added and the mixture stirred for about 30 minutes. The organic layer was separated and washed once more with 10% sodium carbonate solution (1250ml) and once with water (1250ml). The organic layer was dried over anhydrous sodium sulphate, filtered and concentrated under reduced pressure. The concentrate was triturated with toluene (400ml) and the mixture stirred at 0°C for about 1 hour. The resulting solid was filtered, washed with toluene and dried *in vacuo* to give methyl 2-(3-chloropropoxy)-indole-3-carboxylate (245.5g) as a white crystalline solid in 67% yield.

## Claims

1. A process for the preparation of N-[(1-ⁿbutyl-4-piperidinyl)methyl]-3,4-dihydro-2H-[1,3]-oxazino[3,2-a]indole-10-carboxamide (SB 207266) or a pharmaceutically acceptable salt thereof, which process comprises the reaction of of N-(1-ⁿbutyl-4-piperidyl)methylamine with a compound of formula (A): 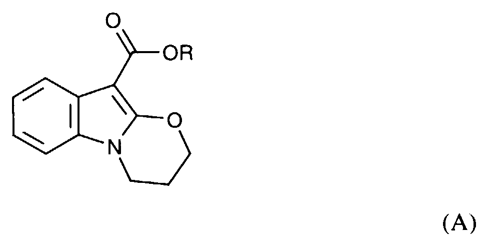 wherein R is alkyl.

2. A process according to claim 1 wherein R is methyl or ethyl.

3. A process according to claim 1 or 2 wherein the reaction is catalysed by an aluminium or lithium based catalyst.

4. A process according to claim 3 wherein the catalyst is trimethylaluminium.

5. A compound of formula (A): 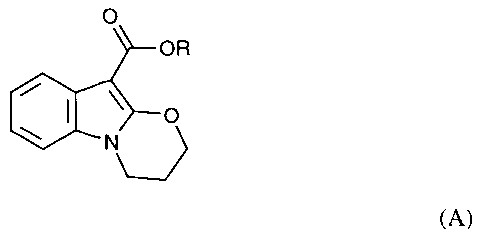 wherein R is alkyl.

6. A compound according to claim 5 which is methyl 3,4-dihydro-2*H*-[1,3]-oxazino[3,2-*a*]indole-10-carboxylate.

7. A compound of formula (B):

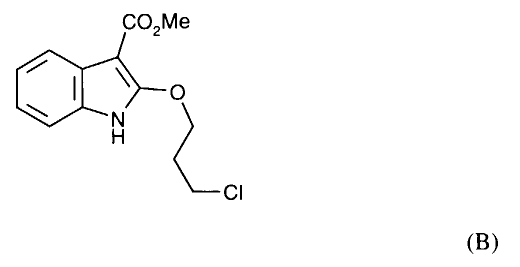

8. A method of preparing methyl 3,4-dihydro-2*H*-[1,3]-oxazino[3,2-*a*]indole-10-carboxylate, comprising cyclising compound (B) as defined in claim 7 by treatment with base in a suitable solvent.

9. A method of preparing N-[(1-ⁿbutyl-4-piperidinyl)methyl]-3,4-dihydro-2*H*-[1,3]-oxazino[3,2-*a*]indole-10-carboxamide (SB 207266) or a pharmaceutically acceptable salt thereof, comprising performing the method of claim 8 and reacting the resulting methyl 3,4-dihydro-2*H*-[1,3]-oxazino[3,2-*a*]indole-10-carboxylate with N-(1-ⁿbutyl-4-piperidyl)methylamine.

## Patentansprüche

1. Verfahren zur Herstellung von N-[(1-n-Butyl-4-piperidinyl)methyl]-3,4-dihydro-2*H*-[1,3]-oxazino[3,2-*a*]indol-10-carbonsäureamid (SB 207266) oder eines pharmazeutisch verträglichen Salzes davon, wobei das Verfahren die Umsetzung von N-(1-n-Butyl-4-piperidyl)methylamin mit einer Verbindung der Formel (A): 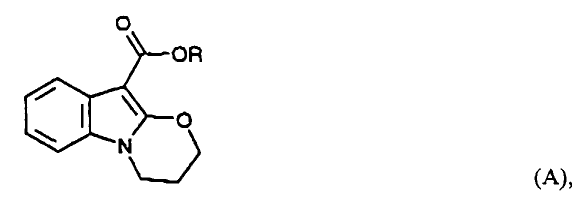 in der R einen Alkylrest bedeutet, umfasst.

2. Verfahren nach Anspruch 1, wobei R eine Methyl- oder Ethylgruppe darstellt.

3. Verfahren nach Anspruch 1 oder 2, wobei die Umsetzung durch einen Katalysator auf Aluminium- oder Lithiumbasis katalysiert wird.

4. Verfahren nach Anspruch 3, wobei der Katalysator Trimethylaluminium ist.

5. Verbindung der Formel (A): 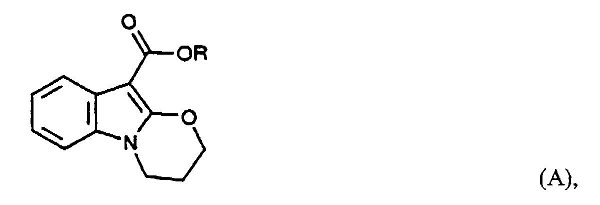 in der R einen Alkylrest bedeutet.

6. Verbindung nach Anspruch 5, und zwar 3,4-Dihydro-2*H*-[1,3]-oxazino[3,2-*a*]indol-10-carbonsäuremethylester.

7. Verbindung der Formel (B):

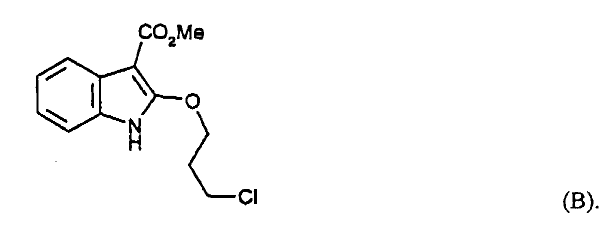

8. Verfahren zur Herstellung von 3,4-Dihydro-2*H*-[1,3]-oxazino[3,2-*a*]indol-10-carbonsäuremethylester, umfassend das Cyclisieren von Verbindung (B) nach Anspruch 7 durch Behandlung mit einer Base in einem geeigneten Lösungsmittel.

9. Verfahren zur Herstellung von N-[(1-n-Butyl-4-piperidinyl)methyl]-3,4-dihydro-2*H*-[1,3]-oxazino[3,2-a]indol-10-carbonsäureamid (SB 207266) oder eines pharmazeutisch verträglichen Salzes davon, umfassend die Durchführung des Verfahrens von Anspruch 8 und die Umsetzung des entstandenen 3,4-Dihydro-2*H*-[1,3]-oxazino[3,2-*a*]indol-10-carbonsäureesters mit N-(1-n-Butyl-4-piperidyl)methylamin.

## Revendications

1. Procédé pour la préparation du N-[(1-ⁿbutyl-4-pipéridinyl)méthyl]-3,4-dihydro-2H-[1,3]-oxazino[3,2-a]indole-10-carboxamide (SB 207 266) ou d'un de ses sels pharmaceutiquement acceptables, procédé qui comprend la réaction de la N-(1-ⁿbutyl-4-pipéridyl)méthylamine avec un composé de formule (A) : 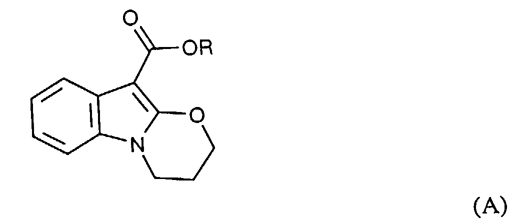 dans laquelle R représente un groupe alkyle.

2. Procédé suivant la revendication 1, dans lequel R représente un groupe méthyle ou éthyle.

3. Procédé suivant la revendication 1 ou 2, dans lequel la réaction est catalysée par un catalyseur à base d'aluminium ou de lithium.

4. Procédé suivant la revendication 3, dans lequel le catalyseur consiste en triméthylaluminium.

5. Composé de formule (A) : 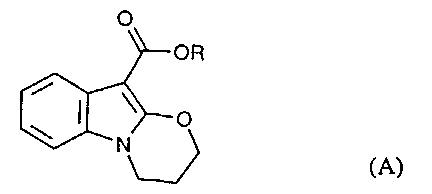 dans laquelle R représente un groupe alkyle.

6. Composé suivant la revendication 5, qui consiste en le 3,4-dihydro-2H-[1,3]oxazino[3,2-a]indole-10-carboxylate de méthyle

7. Composé de formule (B) :

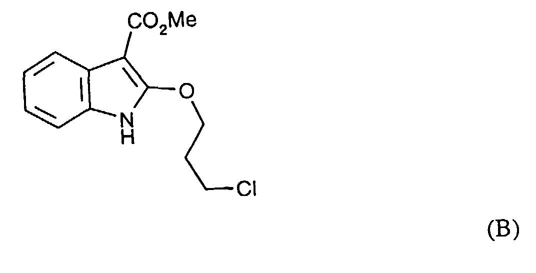

8. Procédé pour la préparation du 3,4-dihydro-2H[1,3]oxazino[3,2-a]indole-10-carboxylate de méthyle, comprenant la cyclisation du composé (B) répondant à la définition suivant la revendication 7 par traitement avec une base dans un solvant convenable.

9. Procédé pour la préparation du N-[(1-ⁿbutyl-4-pipéridinyl)méthyl]-3,4-dihydro-2H-[1,3]-oxazino[3,2-a]-indole-10-carboxamide (SB 207 266) ou d'un de ses sels pharmaceutiquement acceptables, comprenant la mise en oeuvre du procédé suivant la revendication 8 et la réaction du 3,4-dihydro-2H-[1,3]-oxazino[3,2-a]indole-10-carboxylate de méthyle résultant avec la N-(1-ⁿbutyl-4-pipéridinyl)méthylamine.
